# NEUE EUROPÄISCHE PATENTSCHRIFT

(11) **EP 1 047 704 B2**
(45) Veröffentlichungstag und Bekanntmachung des Hinweises auf die Entscheidung über den Einspruch: **07.11.2007**
(45) Hinweis auf die Patenterteilung: 29.01.2003
(21) Anmeldenummer: 98965703.6
(22) Anmeldetag: 30.11.1998
(51) Int. Cl.: C07H 19/00

(54) **VERFAHREN ZUR HERSTELLUNG VON FLUDARABIN-PHOSPHAT-LITHIUM-, NATRIUM-, KALIUM-, CALCIUM- UND MAGNESIUM-SALZEN, REINIGUNGSVERFAHREN ZUR HERSTELLUNG VON FLUDARABIN-PHOSPHAT UND FLUDARABIN-PHOSPHAT MIT MINDESTENS 99,5 %IGER REINHEIT**
METHOD FOR PRODUCING FLUDARABIN PHOSPHATE LITHIUM, SODIUM, POTASSIUM, CALCIUM AND MAGNESIUM SALTS, PURIFICATION METHOD FOR PRODUCING FLUDARABIN PHOSPHATE AND FLUDARABIN PHOSPHATE WHICH IS AT LEAST 99.5 % PURE
PROCEDE DE PREPARATION DE SELS DE LITHIUM, DE SODIUM, DE POTASSIUM, DE CALCIUM ET DE MAGNESIUM DE PHOSPHATE DE FLUDARABINE ET PROCEDE DE PURIFICATION UTILISE POUR PREPARER DU PHOSPHATE DE FLUDARABINE ET PHOSPHATE DE FLUDARABINE A DEGRE DE PURETE D'AU MOINS 99,5%

(30) Priorität: 11.12.1997 DE 19756289
(43) Veröffentlichungstag der Anmeldung: 02.11.2000
(73) Patentinhaber: Bayer Schering Pharma Aktiengesellschaft, 13353 Berlin (DE)
(72) Erfinder: TILSTAM, Ulf, D-13359 Berlin (DE); SCHMITZ, Thomas, D-79639 Grenzach-Wyhlen (DE); NICKISCH, Klaus, D-12307 Berlin (DE)
(74) Vertreter: Weickmann, Heinrich
(86) Internationale Anmeldenummer: PCT/EP1998/007651
(87) Internationale Veröffentlichungsnummer: WO 1999/029710

(56) Entgegenhaltungen:
- DE-A- 19 543 052

## Beschreibung

Die Erfindung betrifft ein Verfahren zur Herstellung von Fludarabin-phosphat Lithium-, Natrium-, Kalium-, Calcium- und Magnesiumsalzen, die als Zwischenprodukte zur Reinigung von FLUDARABIN-PHOSPHAT verwendet werden können.

Fludarabin-phosphat ist der "International Nonproprietary Name" (INN) von 9-β-D-Arabinofuranosyl -2-fluroadenin-5'-O-dihydrogenphosphat. Die erste Synthese der Vorstufe des Fludarabin-phosphat, dem 9-β-D-Arabinofuranosyl-2-fluroadenin, wird in US-PS 4,188,378 beschrieben. Diese Substanz weist stark cytotoxische Eigenschaften auf, und es wurden verschiedene Derivate davon, mit dem Ziel der Reduzierung von Nebenwirkungen, hergestellt. Innerhalb der US-PS 4,357,324 wird das 5 '- Phosphat (Prodrug), also das Fludarabin-phosphat und dessen Herstellung beschrieben. In weiteren Schriften, beispielsweise US-PS 4,210,745, WO 91/08215 und WO 94/12514 werden alternative Herstellungsverfahren offenbart.

Der derzeit benutzte Herstellungsweg geht von 9-β-D-Arabinofuranosyl -2-fluroadenin aus, das mit Trimethylphosphat und Phosphoroxychlorid umgesetzt wird (Phosphorylierung). Diese Edukte werden zur Reaktion gebracht und anschließend aus Wasser kristallisiert. Die bei der Umkristallisation anzuwendende Temperatur von etwa 75 °C zerstört einen Teil der Substanz, da Fludarabin-phosphat bei dieser Temperatur im Wasser thermisch instabil ist. Nachteilig ist weiter, daß diese aus dem Stand der Technik bekannte Umkristallisation nur zu einer schwachen Verbesserung der Reinheit führt und das Verfahren nur in Ansatzgrößen, auch für eine technische Herstellung, von etwa unter 1 kg ausgeführt werden kann. Die innerhalb der DE 41 41 454 A1 beschriebenen Salze des Fludarabin-phosphats sind nach der Lehre dieser Schrift nicht herstellbar. Bei der Anwendung der beschriebenen Reaktionsbedingungen würde es hauptsächlich zu einer Spaltung von Phosphorsäure im Molekül kommen.

In der US 5,296,589 wird in Spalte 10, Zeilen 37 - 40, die Wasserlöslichkeit des Natriumsalzes von Fludarabin-phosphat (2-Fluor-ara-adenosin 5'-phosphat) beschrieben. Weiter wird in Spalte 9, Zeilen 61 - 69, beschrieben, daß sich das Salz durch Umkristallisation aus Wasser nicht reinigen läßt, da diese Bedingungen zur Zersetzung der Verbindung führen würden (siehe auch DE 195 43 052 A1, WO 92/0312 A1 und US 5,506,352).

Aufgabe der vorliegenden Erfindung ist es, ein Reinigungsverfahren bereitzustellen, welches zu einer deutlich verbesserten Qualität (Reinheit) von Fludarabin-phosphat führt und das im großtechnischen Verfahren problemlos auch auf Mengen von über einem Kilogramm angewendet werden kann.

Gelöst wird diese Aufgabe gemäß der Lehre der Patentansprüche.

Die Erfindung betrifft ein Verfahren zur Herstellung von Fludarabin-phosphat Lithium-, Natrium-, Kalium-, Calcium- und Magnesiumsalze , wobei Fludarabinphosphat in Wasser suspendiert, zu dieser Lösung unter Rühren und bei Temperaturen von unterhalb 30 °C eine Alkali- oder Erdalkalibasenlösung gegeben und diese Lösung langsam in 45 - 55 °C warmes Aceton eingefällt, gekühlt und der ausgefallene Niederschlag gegebenenfalls filtriert und gegebenenfalls getrocknet wird und weiter ein Verfahren zur Herstellung von Fludarabin-phosphat, wobei die Lithium-, Natrium-, Kalium-, Calcium- und Magnesiumsalze nach Anspruch 1 hergestellt und anschließend mit Mineralsäure freigesetzt werden.

Als geeignete Basen werden Hydroxide und Carbonate der Alkalien oder Erdalkalien verwendet, die gut in Wasser löslich sind; beispielsweise Lithium-, Natrium-, Kaliumoder Calciumhydroxid; Natrium- oder Kaliumcarbonat.

Wie überraschenderweise gefunden wurde, können Alkali- und Erdalkalisalze des Fludarabin-phosphats als stabile, kristalline und gut charakterisierbare Substanzen hergestellt werden, die sich durch Kristallisation reinigen lassen. Es hat sich gezeigt, daß sich diese Alkali- und Erdalkalisalze des Fludarabin-phosphats problemlos isolieren lassen; auch eine Lagerung über längere Zeit überstehen diese, ohne Instabilitäten zu zeigen. Besonders geeignet sind die Lithium-, Natrium-, Kalium-, Calcium- und Magnesiumsalze.

Hier hat sich mit Vorteil herausgestellt, daß diese Kristallisation besonders gut aus Wasser/Aceton erfolgt. So wird Fludarabin Phosphat durch Zugabe von Natriumcarbonatlösung oder den analogen Basenlösungen der anderen Elemente gelöst und diese wäßrige Lösung in Aceton eingefällt. Beispielsweise werden 6,1 kg Fludarabin-Phosphat in 35 1 Wasser suspendiert; es werden 1,79 kg Natriumcarbonat, gelöst in 7,9 1 Wasser, zugegeben und diese Lösung in 1501 Aceton bei 45 - 55 °C, vorzugsweise bei 50 °C, eingefällt. Die Temperatur darf keinesfalls über 60 °C steigen, da sonst als Nebenreaktion die Substitution von Fluor durch Hydroxyl erfolgt, was unerwünscht ist. Bei der Abkühlung der Mischung bleiben die NICHT- Phosphatierten Derivate in Lösung, das gewünschte Produkt kristallisiert.

Diese Fludarabin-phosphat-Salze der Alkalien und Erdalkalien führen beim Auflösen in Wasser nicht zu stark sauren, sondern zu fast neutralen Lösungen. Die Rückführung dieser Salze in freies Fludarabin-phosphat gelingt durch Versetzen mit starker Mineralsäure ohne Schwierigkeiten. Als Mineralsäuren finden beispielsweise Salzsäure, Schwefelsäure, Salpetersäure oder Phosphorsäure Anwendung. Bei der Freisetzung der freien Base bleiben die MEHRFACH-phosphatierten Nebenprodukte in Lösung.

Die Salze des Fludarabin-phosphats können als Vorstufen von Fludarabin-phosphat problemlos über längere Zeit gelagert werden und im Bedarfsfall der Wirkstoff freigesetzt werden.

Die Erfindung betrifft auch ein Verfahren zur Herstellung von Fludarabin-phosphat mit einer Reinheit von mindestens 99,5 %. Gemäß des Standes der Technik konnte der Wirkstoff bisher nur in einer Reinheit von etwa 98,0 - 98,5 % erhalten werden. Es ist unmöglich, durch die herkömmlichen Kristallisationsverfahren, beispielsweise aus Wasser, einen Reinheitsgrad von 98,5 % zu überschreiten; auch dann, wenn mehrfach dieselbe Charge kristallisiert wird. Problematisch ist diese herkömmliche Reinigungsmethode dann, wenn die Zeit für das Aufheizen und Filtration der beispielsweise wäßrigen Lösung zu lange Zeit benötigt; dies sind Zeiten von 25 min. und mehr. Es kommt in diesem Fall zur Bildung von diversen Verunreinigungen, gummiartigen Massen, die sich nicht mehr durch Kristallisationsmethoden entfernen lassen.

Durch das erfindungsgemäße Verfahren lassen sich Fludarabin-phosphat Reinheiten beispielsweise von 99,5; 99,55; 99,6; 99,65; 99,7; 99,75; 99,8; 99,9 oder 99,95 % erhalten; bereits dann, wenn Fludarabin-phosphat nur einmal nach dem erfindungsgemäßen Verfahren gereinigt wird. Es ist aber auch möglich, das Verfahren für eine Fludarabin-phosphat-Charge zwei- und mehrfach anzuwenden.

Die folgenden Beispiele sollen die Erfindung näher erläutern:

### Beispiel 1

### FLUDARABIN-PHOSPHAT-DINATRIUMSALZ

5,0 g Fludarabin-phosphat in einer Reinheit von 98,5 % werden in 30 ml Wasser suspendiert und etwa 3 - 5 Minuten gerührt. Zu dieser Suspension werden unter Rühren und bei Temperaturen von unterhalb 30 °C 6,5 ml einer Sodalösung (18,5 Gewichtsprozent) gegeben. Nach beendeter Zugabe wird die Mischung 15 Minuten gerührt und anschließend vom ungelösten Material filtriert. Die so erhaltene klare Lösung wird langsam in Aceton (bei 50 °C) eingefällt. Es wird 2 Stunden nachgerührt und gekühlt. Der ausgefallene Niederschlag wird filtriert, mit Aceton gewaschen und getrocknet; es werden 5,0 g des Fludarabin-phosphats dinatriumsalz erhalten, 98 % der Theorie.
Schmelzpunkt: 235 °C; Reinheit: 98,5 %
Analyse: Ber., für C₁₀H₁₁FNa₂N₅O₇Px2H₂O (445,20)
C, 26,98; H, 3,39; F, 4,27; N, 15,73; P, 6,96; Na10,33
Gefunden: C 27,15; H 3,93; N15,72; Na 9,65; P 6,15
IR (KBr): 3420, 3340, 3200, 2910, 1650, 1610, 1390, 1210, 1100, and 980 cm⁻¹. NMR (D₂O): 4,05-4,22 m (3H; H-4'; both H-5'); 4,45-4,60 m (2H; H-2'und H-3'); 6,2 d (1H; H-1'); 8,45 s (1H; H-8).

### Beispiel 2

### FREISETZUNG VON FLUDARABIN-PHOSPHAT AUS DEM DINATRIUMSALZ

5,0 g Fludarabin-phosphat Dinatriumsalz nach Beispiel 1 werden innerhalb von 3 - 5 Minuten in 35 ml Wasser gelöst. Die Lösung wird filtriert, langsam mit 5 ml Salzsäure (37 %ig) versetzt und 1 - 2 Stunden gerührt. Der ausgefallene Niederschlag wird abgesaugt, mit Eiswasser und Ethanol gewaschen und ergibt 4,0 g Fludarabinphosphat, 90 % der Theorie.
Schmelzpunkt 202 - 203 °C; Reinheit: 99,6 %
Analyse, Ber.: C₁₀H₁₃FN₅O₇P (365,21)
C, 32,89; H, 3,59; N, 19,17; F, 5,20; P, 8,48
Gefunden:.: C, 32,81; H, 3,65; N, 19,03; P, 8,41
IR (KBr): 3443, 3326, 3123, 2925, 2950-2100, 1663, 1591, 1211, 1126, und 1050 cm^{-1.}

NMR (DMSO): 3,94-3,99 m (1H; H-4'); 4,06-4,14 m (3H; H-3'; both H-5'); 4,14-4,18 m (1H; H-2'); 5,4-6,1 broad (OH protons); 6,17 d (1H; H-1'); 7,6-8,0 broad (NH-protons); 8,14 s (1H; H-1') 9-11 broad (P-OH).

### Beispiel 3

### FLUDARABIN-PHOSPHAT DILITHIUMSALZ

10,0 g Fludarabin-phosphat in einer Reinheit von 97,4 % werden in 70 ml Wasser innerhalb von etwa 5 Minuten suspendiert und mit einer wäßrigen Lithium-Hydroxydlösung (10 %) versetzt. Diese Lösung wird eine Stunde bei Raumtemperatur gerührt und anschließend filtriert. Die so erhaltene klare Lösung wird in 250 ml Aceton (bei 50 °C) eingefällt und 1 Stunde nachgerührt. Der ausgefallene Niederschlag wird filtriert, mit Aceton gewaschen und ergibt nach Trocknung 4,3 g des Fludarabin-phosphat-dilithiumsalz. (90 % der Theorie).
Schmelzpunkt 240 - 260 °C, Reinheit: 98,5 %
Analyse: Ber.:für C₁₀H₁₁FLi₂N₅O₇P x 3H₂O(431,12)
C, 27,86; H, 3,98; F, 4,41; N, 16,25; P, 7,18; Li,3,22
Gef.: C 27,15; H 3,86; N15,76; Li, 3,05; P 6,72
NMR (D₂O):4,05-4,22 m (H-4';H-5'); 4,45 -4,55 m( H-2'und H-3'); 6,25 d (H-1'); 8,50 s (H-8).

Die Freisetzung gemäß dem Beispiel 2 führt zu einer Fludarabin-phosphat Reinheit von 99,85%.

### Beispiel 4

### FLUDARABIN-PHOSPHAT - DIKALIUMSALZ

5,0 g Fludarabin-phosphat in einer Reinheit von 96,1 % werden in 30 ml Wasser gelöst; zu dieser Lösung werden unterhalb von 30 °C 6,5 ml einer Kaliumcarbonatlösung (18,5 Gew. % ) zugegeben. Es wird 15 Minuten nachgerührt, danach vom festen Material filtriert. Die so erhaltene klare Lösung wird bei 50° in Aceton eingefällt, auf Raumtemperatur abgekühlt und 2 Stunden nachgerührt. Der ausgefallene Niederschlag wird filtriert und zweimal mit Aceton gewaschen.
Erhalten werden 4,5 g Fludarabin-phosphat Dikaliumsalz.
Schmelzpunkt 220 - 230 °C, Reinheit: 98,55 %.
IR (KBr): 3420, 3340, 3200, 2910, 1650, 1610, 1390, 1210, 1100, und 980 cm⁻¹.

NMR (D₂O):4,0-4,2 m (H-4';H-5'); 4,4 -4,60 m (H-2' und H-3'); 6,25 d (H-1 '); 8,5 s ( H-8).

Die Freisetzung gemäß dem Beispiel 2 führt zu einer Fludarabin-phosphat Reinheit von 99,80 %.

### Beispiel 5

### FLUDARABIN-MAGNESIUMSALZ

10,0 g Fludarabinphosphat in einer Reinheit von 97,5 % werden in 100 ml Wasser innerhalb von etwa 5 Minuten suspendiert; zu dieser Lösung wird Magnesiumoxyd gegeben. Die Mischung wird eine Stunde bei Raumtemperatur nachgerührt und anschließend filtriert. Die klare Lösung wird in 200 ml Aceton eingerallt, 1 Stunde nachgerührt und das Kristallisat durch Filtration getrennt. Es werden 10, 0 g (95 % der Theorie) des Fludarabin-phosphat Magnesiumsalz erhalten.
Schmelzpunkt: 260 °C, Reinheit: 98,45 %
Analyse: Ber. für C₁₀H₁₁FMgN₅O₇P x2H₂O(423,525)
C, 28,36; H, 3,57; F, 4,49; Mg, 5,74; N, 16,54; P, 7,31
Gefunden: C, 27,99; H, 3,92; Mg, 5,54; N, 16,38;
IR (KBr): 3420, 3340, 3200, 2910, 1650, 1610, 1390, 1210, 1100, and 980 cm⁻¹.

NMR (D₂O):4,0-4,2 m (H-4';H-5'); 4,5 -4,60 m (H-2'und H-3'); 6,2 d (H-1'); 8,4 s (H-8).

Die Freisetzung gemäß dem Beispiel 2 führt zu einer Fludarabin-phosphat Reinheit von 99,55%.

## Patentansprüche

1. Verfahren zur Herstellung von Fludarabin-phosphat Lithium-, Natrium-, Kalium-, Calcium- und Magnesiumsalzen, wobei Fludarabin-phosphat in Wasser gelöst, zu dieser Lösung unter Rühren und bei Temperaturen von unterhalb 30 °C eine Alkali- oder Erdalkalibasenlösung gegeben und diese Lösung langsam in 45-55 °C warmes Aceton eingefällt, gekühlt und der ausgefallene Niederschlag gegebenenfalls filtriert und gegebenenfalls getrocknet wird.

2. Verfahren zur Herstellung von Fludarabin-phosphat, wobei die Lithium-, Natrium-, Kalium-, Calcium- und Magnesiumsalze nach Anspruch 1 hergestellt und anschließend mit Mineralsäure freigesetzt werden.

3. Verfahren zur Herstellung von Fludarabin-phosphat, wobei die Lithium-, Natrium-, Kalium-, Calcium- und Magnesiumsalze in lagerstabilerer Form nach Anspruch 1 hergestellt und anschließend mit Mineralsäure freigesetzt werden.

4. Verfahren zur Reinigung von Fludarabin-phosphat, wobei rohes Fludarabin-phosphat in Wasser gelöst, zu dieser Lösung: unter Rühren und bei Temperaturen von unterhalb 30 °C- eine Alkali- oder Erdalkalibasenlösung gegeben und diese Lösung langsam in 45-55 °C warmes Aceton eingefällt, gekühlt und der ausgefallene Niederschlag filtriert und gegebenenfalls getrocknet und in einer lagerstabilen Form als Lithium-, Natrium-, Kalium-, Calcium- oder Magnesiumsalze erhalten wird und anschließend diese lagerstabile Form in Wasser gelöst und mit Mineralsäure angesäuert und der ausgefällte Niederschlag filtriert und getrocknet wird.

5. Verfahren nach einem der Ansprüche 2 bis 4,
**dadurch gekennzeichnet,**
**dass** ein Fludarabin-phosphat mit einer Reinheit von mindestens 99,5 % erhalten wird.

6. Verfahren nach einem der Ansprüche 2 bis 4,
**dadurch gekennzeichnet,**
**dass** ein Fludarabin-phosphat mit einer Reinheit von größer 99,55 % erhalten wird.

7. Verfahren nach einem der Ansprüche 2 bis 4,
**dadurch gekennzeichnet,**
**dass** ein Fludarabin-phosphat mit einer Reinheit von größer 99,6 % erhalten wird.

8. Verfahren nach einem der Ansprüche 2 bis 4,
**dadurch gekennzeichnet,**
**dass** ein Fludarabin-phosphat mit einer Reinheit von größer 99,7 % erhalten wird.

9. Verfahren nach einem der Ansprüche 2 bis 4,
**dadurch gekennzeichnet,**
**dass** ein Fludarabin-phosphat mit einer Reinheit von größer 99,8 % erhalten wird.

10. Verfahren nach einem der Ansprüche 2 bis 4,
**dadurch gekennzeichnet,**
**dass** ein Fludarabin-phosphat mit einer Reinheit von größer 99,85 % erhalten wird.

## Claims

1. Process for the production of fludarabine phosphate lithium, sodium, potassium, calcium and magnesium salts, where fludarabine phosphate is dissolved in water, an alkali or alkaline-earth basic solution is added to this solution while being stirred and at temperatures of below 30°C, and this solution is slowly poured into acetone that is 45-55°C, cooled, and the deposited precipitate is optionally filtered and optionally dried.

2. Process for the production of fludarabine phosphate, where the lithium, sodium, potassium, calcium and magnesium salts are produced according to Claim 1 and then are released with mineral acid.

3. Process for the production of fludarabine phosphate, where the lithium, sodium, potassium, calcium and magnesium salts are produced in a form that is more stable in storage according to Claim 1 and then are released with mineral acid.

4. Process for the purification of fludarabine phosphate, where crude fludarabine phosphate is dissolved in water, an alkali or alkaline-earth basic solution is added to this solution while being stirred and at temperatures of below 30°C, and this solution is slowly poured into acetone that is 45-55°C, cooled, and the deposited precipitate is filtered and optionally dried and is obtained in a form that is stable in storage as a lithium, sodium, potassium, calcium or magnesium salt, and then this form that is stable in storage is dissolved in water and acidified with mineral acid, and the deposited precipitate is filtered and dried.

5. Process according to any one of claims 2 to 4, **characterised in that** fludarabine phosphate with a purity of at least 99.5% is obtained.

6. Process according to any one of claims 2 to 4, **characterised in that** fludarabine phosphate with a purity of more than 99.55% is obtained.

7. Process according to any one of claims 2 to 4, **characterised in that** fludarabine phosphate with a purity of more than 99.6% is obtained.

8. Process according to any one of claims 2 to 4, **characterised in that** fludarabine phosphate with a purity of more than 99.7% is obtained.

9. Process according to any one of claims 2 to 4, **characterised in that** fludarabine phosphate with a purity of more than 99.8% is obtained.

10. Process according to any one of claims 2 to 4, **characterised in that** fludarabine phosphate with a purity of more than 99.85% is obtained.

## Revendications

1. Procédé de préparation de sels de lithium, de sodium, de potassium, de calcium et de magnésium de phosphate de fludarabine, le phosphate de fludarabine étant dissous dans de l'eau, une solution de base alcaline ou alcalino-terreuse étant ajoutée à cette solution sous agitation et à des températures inférieures à 30°C et cette solution étant versée lentement dans de l'acétone chaude à 45-55°C, refroidie et le précipité formé étant le cas échéant filtré et le cas échéant séché.

2. Procédé de préparation de phosphate de fludarabine, les sels de lithium, de sodium, de potassium, de calcium et de magnésium étant préparés selon la revendication 1 et ensuite libérés avec un acide minéral.

3. Procédé de préparation de phosphate de fludarabine, les sels de lithium, de sodium, de potassium, de calcium et de magnésium étant préparés sous forme stable à l'entreposage selon la revendication 1 et ensuite libérés avec un acide minéral.

4. Procédé de purification de phosphate de fludarabine, du phosphate de fludarabine brut étant dissous dans de l'eau, une solution de base alcaline ou alcalino-terreuse étant ajoutée à cette solution sous agitation et à des températures inférieures à 30° C et cette solution étant versée lentement dans de l'acétone chaude à 45-55°C, refroidie et le précipité formé étant filtré et le cas échéant séché et obtenu sous une forme stable à l'entreposage sous forme de sels de lithium, de sodium, de potassium, de calcium ou de magnésium et cette forme stable à l'entreposage étant dissoute dans de l'eau et acidifiée avec un acide minéral et le précipité formé étant filtré et séché.

5. Procédé selon l'une quelconques des revendications 2 à 4, **caractérisé en ce qu'**il est obtenu un phosphate de fludarabine présentant une pureté d'au moins 99,5%.

6. Procédé selon l'une quelconques des revendications 2 à 4, **caractérisé en ce qu'**il est obtenu un phosphate de fludarabine présentant une pureté supérieure à 99,55%.

7. Procédé selon l'une quelconques des revendications 2 à 4, **caractérisé en ce qu'**il est obtenu un phosphate de fludarabine présentant une pureté supérieure à 99,6%.

8. Procédé selon l'une quelconques des revendications 2 à 4, **caractérisé en ce qu'**il est obtenu un phosphate de fludarabine présentant une pureté supérieure à 99,7%.

9. Procédé selon l'une quelconques des revendications 2 à 4, **caractérisé en ce qu'**il est obtenu un phosphate de fludarabine présentant une pureté supérieure à 99,8%.

10. Procédé selon l'une quelconques des revendications 2 à 4, **caractérisé en ce qu'**il est obtenu un phosphate de fludarabine présentant une pureté supérieure à 99,85%.
